# EUROPEAN PATENT APPLICATION

(11) **EP 1 142 577 A2**
(43) Date of publication of application: **10.10.2001**
(21) Application number: 01302485.6
(22) Date of filing: 19.03.2001
(51) Int. Cl.: A61K 31/7048, A61P 33/00, A01N 43/90

(54) **Method of treating a parasitic infection**

(30) Priority: 03.04.2000 GB 0008353
(71) Applicant: Pfizer Limited, Sandwich, Kent CT13 9NJ (GB); PFIZER INC., New York, N.Y. 10017 (US)
(72) Inventor: Benchaoui, Hafid Abdelaali, Pfizer Global Res., Sandwich, Kent CT13 9NJ (GB); Jernigan, Antoinette Beaton Drain, Groton, Connecticut 06340 (US); Payne-Johnson, Mark, Pfizer Global Res. and Dev., Sandwich, Kent CT13 9NJ (GB); Rowan, Timothy George, Pfizer Global Res. and Dev., Sandwich, Kent CT13 9NJ (GB)
(74) Representative: Ruddock, Keith Stephen

(57) **Abstract**

When selamectin is administered to a female animal over a period commencing during pregnancy and ending during lactation, endo- and ecto-parasite infestations are either treated or prevented in that animal's offspring, avoiding the problems of treating the offspring directly. This leads to improvements in the compliance of dosing and hence the efficacy of treatment and reduces the number of doses to be administered leading to economic benefits and greater convenience.

## Description

This invention relates to a new use of selamectin.

The antiparasitic activity of selamectin, a compound structurally related to the avermectins and milbemycins, was disclosed in International Patent Application WO-94/15944 (see Example 5). Selamectin has the following structure:

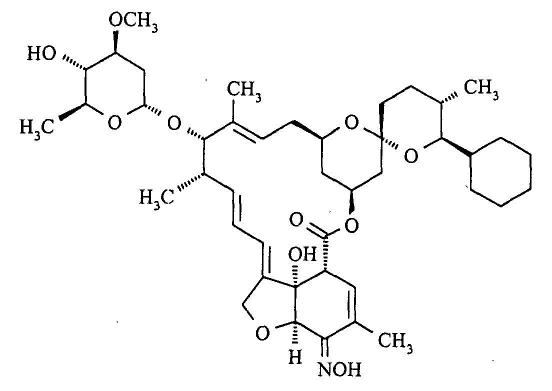

Selamectin has proved successful in the control of a range of parasitic infections in companion animals, especially in cats and dogs. It is described as a companion animal endectocide, being effective in the control of both endoparasites such as intestinal nematodes and ectoparasites such as fleas. Conventional disaccharide avermectins lacking an oxime group, such as doramectin and ivermectin, are not as useful as companion animal endectocides since they have poor activity against ectoparasites such as fleas.

It is known that ivermectin can prevent intestinal nematode infections in pups when administered to a pregnant bitch over a period starting in pregnancy and extending past parturition (see *Veterinary Parasitology,* 1999, **85,** 305).

Whilst the use of selamectin has been a great success in the treatment and prophylaxis of parasite infections in companion animals, its use in very young animals such as puppies and kittens less than six weeks of age has been limited by a number of factors and the latter use is not currently licensed. Conventional antiparasitic treatment regimens for young animals require regular repeat dosing and such a dosing regime leads to poor compliance and the likelihood of overdosing. This is especially the case since whichever means of administration is chosen (e.g. oral, topical, injection) it is much harder to deliver the correct dose, accurately, to a small mammal.

It has now been found that when selamectin is administered to a female animal over a period commencing during pregnancy and ending during lactation, endo-and ecto-parasite infestations are either treated or prevented in that animal's offspring, avoiding the problems of treating the offspring directly. This leads to improvements in the compliance of dosing and hence the efficacy of treatment and reduces the number of doses to be administered leading to economic benefits and greater convenience.

Derivatives of selamectin that have antiparasitic activity may equally be used in the invention. Preferred derivatives are solvates and esters. Avermectins and compounds of similar structure are well known to form solvates (see, for example, WO-A-95/10525 and WO-A-99/07721) and other forms of derivatisation are conventional in the art (see, for example, EP-A-0379341).

Thus, according to the present invention, there is provided the use of selamectin, or a derivative thereof, in the manufacture of a medicament for the treatment and prevention of parasite infestations in and on a young animal, said medicament being administered to the female parent of the young animal over a period commencing before, and ending after, parturition.

The invention further provides a method of treating and preventing parasite infestations in and on a young animal, which comprises administering a therapeutically effective amount of selamectin, or a derivative thereof, to the female parent of that animal over a period commencing before, and ending after, parturition.

For the purposes of the invention an animal may be defined as young if it is at an age where it would usually gain part of its sustenance from suckling.

Animals which may be treated according to the invention include companion animals and livestock, particularly companion animals such as cats, dogs, rabbits, ferrets and rats. The invention is particularly useful in the treatment of cats and dogs.

Parasites which may be controlled by the invention include adult and immature fleas (*Ctenocephalides felis and Ctenocephalides canis),* ear mites (*Otodectes cynotis*), mange mites (*Sarcoptes scabiei*, *Cheyletiella spp.),* lice of dogs and cats (*Trichodectes canis*, *Linognathus setosu* and *Felicola subrostratus*), ticks (e.g. *Rhipicephalus sanguineus* and other ixodid ticks), roundworm (*Toxocara canis* and *Toxocara cati*), heartworm larvae (*Dirofilaria immitis*), and hookworm (*Ancylostoma tubaeforme).*

Parasites which are particularly well controlled by the invention are fleas, mites, lice, hookworm and roundworm.

Selamectin may be administered to the female animal whose offspring are to be treated according to the invention by any conventional route, e.g. topically, orally or by injection. It may be administered alone but will more conventionally be administered in admixture with a pharmaceutically acceptable diluent or carrier. Suitable formulations and routes are well known to the skilled man and are illustrated by the Examples below and by the disclosure of WO-A-94/15944.

According to the present invention, selamectin should preferably be dosed to the adult female animal during the period of six weeks before parturition and 6 weeks after parturition. Doses will typically be administered at intervals of 20-30 days. For example, a suitable dosing regime would be 6 weeks and 2 weeks *pre-partum* and 2 weeks and 6 weeks *post-partum.*

The amount of drug administered at each dosing point will vary with the size of the animal, the severity of parasite challenge and the type of parasite against which protection is sought. It will typically be in the range 1-60 mg/kg, preferably 6-12 mg/kg, for topical administration. For treating fleas and roundworm in cats and dogs, the most preferred dose is 6 mg/kg applied topically.

According to the present invention, selamectin may be administered in combination with another antiparasitic agent, for example amitraz.

The following Examples are illustrative of the invention.

### Example 1

### Determination of the efficacy of selamectin administered to pregnant/lactating female dogs against Toxocara Canis and Ctenocephalides fells in their offspring

### Experimental Method

Twenty-one adult females were allocated randomly to one of two treatments: placebo (negative control, 11 animals), or selamectin (10 animals). The selamectin, was formulated as:
120.00 mg/ml selamectin in:

| | |
|---|---|
| Dipropylene glycol monomethyl ether | 112.56 mg/ml |
| Butylhydroxytoluene | 0.80 mg/ml |
| Isopropyl alcohol | 613.64 mg/ml |

The placebo solution was similar, containing dipropylene glycol monomethyl ether, butylhydroxytoluene and isopropyl alcohol, but without selamectin.

Doses providing 6 mg/kg of selamectin were administered topically approximately 40 days and 10 days *pre-partum* and 10 days and 38 days *post-partum* in a single spot on the skin of each female's back at the base of the neck in front of the shoulder blades. Treatments were not administered to puppies. *Post-partum* day 1 was defined individually for each adult female as the day following the day of delivery of the last puppy in the litter.

All adult females selected for the study had been shown to be capable of carrying a flea burden after infestation with C. *felis,* were known to have a satisfactory breeding history, and came from a group-housed breeding colony with long-established endemic *T. canis* infection, as shown by routine *Tcanis* faecal egg counts. Following observations of signs of pro-oestrus, the females were moved into randomly allocated carpeted pens in a building where the environment was suitable for the rapid completion of the flea reproductive cycle.

Each adult female was mated to one of four randomly allocated male dogs, and pregnancy was diagnosed by ultrasonic examination and palpation approximately 32 days and 29 days *pre-partum*.

Each adult female was infested with 100 viable unfed adult *C. felis* approximately 60 days and 46 days *pre-partum*, and with 10 viable unfed adult fleas approximately 39, 33, 26, 19, 14 and 5 days *pre-partum*; an additional infestation was performed 7 days later if the female had not whelped. Each lactating female was also infested with 10 fleas on *post-partum* days 6, 15, 21, 28, 34 and 42. Comb counts of the number of viable adult fleas present on each female were made on approximately *pre-partum* days 41 and 11, and on *post-partum* days 9, 37 and 45. Comb counts of the fleas present on each puppy were performed on *post-partum* days 15, 24, 34, 42 and 45. After counting, up to 50 fleas per pregnant/lactating female and up to five fleas per puppy were replaced on each dog. For welfare reasons, if any dog was scratching or grooming excessively, additional unscheduled comb counts were performed on that dog and if necessary, the carpet was partially or entirely replaced to reduce the environmental flea challenge. Faecal egg counts for *T*. *canis* were performed for pregnant/lactating females on approximately *pre-partum* day 2 and on *post-partum* days 9, 17, 24 and 45, and for puppies on post-partum days 24 and 34.

On *post-partum* day 45, each puppy was euthanised, its gastro-intestinal tract was removed and intestinal larval (L4) and adult *T. canis* were enumerated.

### Results

Of the 21 adult females enrolled in the study, 19 whelped and 17 completed the study with their litters (nine placebo-treated animals and eight selamectin-treated animals). In these dogs, various clinical conditions commonly associated with colony-housed dogs and breeding females (for example, complications at parturition, metritis, skin ulceration and redness, trauma) were diagnosed which generally resolved satisfactorily after appropriate antimicrobial therapy. Of the 120 pups born (63 from placebo-treated females and 57 from selamectin-treated females), 78 completed the study (37 from placebo-treated females and 41 from selamectin-treated females). Puppies failed to complete the study for reasons commonly associated with breeding, for example, stillbirths, maternally induced trauma, unthriftiness and weakness at birth and congenital abnormalities. One placebo-treated female, diagnosed as having metritis, was withdrawn with her litter on welfare grounds when her condition deteriorated despite appropriate antimicrobial and supportive therapy. In addition, one pup had an episode of enteritis and a number of other pups in both treatments had mild skin conditions; these responded satisfactorily to antimicrobial therapy and the affected pups completed the study satisfactorily.

### Faecal egg counts

On *post-partum* days 24 and 34 geometric mean faecal egg counts were 23.7 and 52.3 epg, respectively, for puppies born to placebo-treated females and 0.8 and 2.1 epg, respectively, for puppies born to selamectin-treated females. Puppies born to selamectin-treated females showed reductions in the mean faecal egg counts of 96.8% and 96.1% on *post-partum* days 24 and 34 respectively, compared with puppies born to placebo-treated females.

### Intestinal worm counts

All roundworms found in the intestinal contents of puppies at necropsy on *post-partum* day 45 were identified as *T. canis* adults. Worms were present in at least one puppy in each of the nine litters from placebo-treated females which completed the study. Geometric mean adult *T. canis* worm counts were 3.0 and 0.1, for puppies born to placebo- and selamectin-treated females respectively, and puppies born to selamectin-treated females showed a reduction in mean adult *T*. *canis* count at necropsy on *post partum* day 45 of 98.2%, compared with puppies born to placebo-treated females.

### Flea counts

On *post-partum* days 15, 24, 34, 42 and 45, fleas were observed on all puppies born to placebo- treated females, geometric mean flea comb counts on these days being 53.3, 68.5, 82.3, 48.8 and 38.8, respectively. For puppies born to selamectin-treated females, geometric mean flea comb counts were 0 on all counting days. Compared with puppies born to placebo-treated females, puppies born to selamectin-treated females showed reductions in mean flea comb counts of 100.0% on all counting days.

### Additional flea control measures

None of the selamectin-treated females or their puppies required additional flea control measures to maintain flea burdens within acceptable limits. Of the nine placebo-treated females and their litters which completed the study, eight females and/or their puppies required additional flea control measures on welfare grounds due to large flea infestations after day 0. These additional flea control measures consisted of one or more additional unscheduled flea combings for the female and/or puppies, and also, for seven females and their litters, at least one replacement of the infested carpet in the pen.

### Conclusion

Topical administration of 6 mg/kg of selamectin at monthly intervals to female dogs during pregnancy (with a dose approximately ten days before parturition) and at approximately monthly intervals commencing ten days *post-partum* was safe and highly effective in the treatment and prevention of both *T*. *canis* infections and *C. felis* infestations in pregnant/lactating females and in their nursing puppies for approximately seven weeks.

### Example 2

### Determination of the efficacy of selamectin administered to pregnant/lactating female cats against Toxocara Cati and Ctenocephalides felis in their offspring

### Experimental Method

Thirty-eight adult females were allocated randomly to one of two treatments: placebo (negative control, 19 animals), or selamectin (19 animals). The selamectin, was formulated as:
60.00 mg/ml selamectin in:

| | |
|---|---|
| Dipropylene glycol monornethyl ether | 56.28 mg/ml |
| Butylhydroxytoluene | 0.80 mg/ml |
| Isopropyl alcohol | 697.92 mg/ml |

The placebo solution was similar, containing dipropylene glycol monomethyl ether, butylhydroxytoluene and isopropyl alcohol, but without selamectin.

Doses providing 6 mg/kg of selamectin were administered topically in a single spot on the skin of each female's back at the base of the neck in front of the shoulder blades on days 0 and 30 to individually-housed females and to females group-housed for mating on days 0, 30, 60 and 90 as required until kittening. All females were treated on *post partum* days 10 and 38. Treatments were not administered to kittens. Treatments were not administered to puppies. Study day 0 was defined individually for each adult female as the day on which the first treatment with either placebo or selamectin was administered and *post partum* day 1 was defined as the day following the day of delivery of the last kitten in the litter.

During mating, adult females were either individually or group-housed, and they were moved into the facilities on day -23 (individually-housed cats) or day -5 (group-housed cats).

Each adult female was inoculated once with approximately 1000 embryonated T. *cati* ova, *per os,* between 150 and 31 days prior to commencing the study and again between 5 and 21 days before kittening.

All adult females selected for the study had been shown to be capable of carrying a flea burden after infestation with C. *felis* and were known to have a satisfactory breeding history. Adult females were mated to randomly selected male cats and pregnancy was diagnosed by multiple ultrasound examinations. Females group-housed for mating were moved to individual rooms following confirmation of pregnancy. Rooms used to house pregnant and lactating females and their litters contained carpeting and the environment was suitable for the rapid completion of the flea reproductive cycle.

Those females individually-housed for mating were infested with 100 viable unfed adult *C. felis* on days -20 and -14. Following confirmation of pregnancy, females group-housed for mating were introduced to a flea infested environment and infested with 10 viable unfed adult *C. felis.* All adult females were further infested with 10 fleas at approximately weekly intervals throughout pregnancy and on *post partum* days 6, 15, 21, 28, 34 and 42. Comb counts of the number of viable adult fleas present on each female were performed on *post partum* days 9, 37 and 49 and for kittens on *post partum* days 15, 24, 34, 42 and 49. After counting, up to 50 fleas per adult female and up to five fleas per kitten were replaced on each cat.

For welfare reasons, if any cat was scratching or grooming excessively, additional unscheduled comb counts were performed on that cat and if necessary, the carpet was partially or entirely replaced to reduce the environmental flea challenge. Faecal samples were obtained from lactating females on *post partum* days 9, 24 and 49 for determination of faecal *T. cati* egg counts.

On *post partum* day 49, each kitten was euthanased, its gastro-intestinal tract was removed and intestinal larvae (L4) and adult *T. cati* were enumerated.

### Results

### Intestinal worm counts

At necropsy on *post partum* day 49, adult *T. cati* worms were found in the intestinal contents of kittens from each of the eight litters born to placebo-treated females which completed the study, and *T. cati* fourth stage larvae (L4) were found in kittens from seven of these eight litters. No L4 or adult *T. cati* were found in any kittens born to selamectin-treated females at *post partum*. day 49. Geometric mean L4 and adult *T. cati* counts were 1.1 and 5.4, respectively, for kittens born to placebo-treated females, and 0.0 and 0.0, respectively, for kittens born to selamectin-treated females. Kittens born to selamectin-treated females showed a reduction in mean fourth stage larvae (L4) and adult *T. cati* counts on *post partum* day 49 of 100%, compared with kittens born to placebo-treated females.

### Flea counts

On *post partum* days 24, 34, 42 and 49, fleas were observed on all kittens born to placebo- treated females and geometric mean flea comb counts on *post partum* days 15, 24, 34, 42 and 49 were 12.4, 19.2, 22.7, 21.7 and 35.8, respectively. For kittens born to selamectin-treated females, geometric mean flea comb counts were zero on all counting days. Compared with kittens born to placebo-treated females, kittens born to selamectin-treated females showed reductions in mean flea comb counts of 100% on all counting days.

### Additional flea control measures

After the first treatment on day 0, none of the selamectin-treated females or their kittens required additional flea control measures to maintain flea burdens within acceptable limits. Of the ten placebo-treated females which completed the study, seven adult females and/or their kittens required additional flea control measures on welfare grounds due to large flea infestations after day 0. These additional flea control measures consisted of one or more additional unscheduled flea combings for the female and/or kittens, and also, for three females and their litters, at least one complete replacement of the infested carpet in the room.

### Conclusion

Topical administration of 6 mg/kg of selamectin at monthly intervals to female cats during pregnancy and at monthly intervals commencing ten days *post partum* was safe and highly effective in the treatment and prevention of *C. felis* infestations on, and *T*. *cati* infections in, the nursing kittens for the first seven weeks of life.

## Claims

1. The use of selamectin, or a derivative thereof, in the manufacture of a medicament for the treatment and prevention of parasite infestations in and on a young animal, said medicament being administered to the female parent of the young animal over a period commencing before, and ending after, parturition.

2. The use of claim 1 wherein the animal is a cat or dog.

3. The use of claim 1 or claim 2 wherein the parasites controlled are roundworm, hookworm, mites, lice or fleas.

4. The use of any of claims 1 to 3 wherein 6 mg/kg of selamectin is dosed topically to the female parent at time points 6 weeks and 2 weeks *pre-partum* and 2 weeks and 6 weeks *post-partum*.

5. A method of treating and preventing parasite infestations in and on a young animal, which comprises administering a therapeutically effective amount of selamectin, or a derivative thereof, to the female parent of that animal over a period commencing before, and ending after, parturition.

6. The method of claim 5 wherein the animal is a cat or dog.

7. The method of claim 5 or claim 6 wherein the parasites controlled are roundworm, hookworm, mites, lice or fleas.

8. The method of any of claims 5 to 7 wherein 6 mg/kg of selamectin is dosed topically to the female parent at time points 6 weeks and 2 weeks *pre-partum* and 2 weeks and 6 weeks *post-partum*.
